# EUROPÄISCHE PATENTSCHRIFT

(11) **EP 3 454 830 B1**
(45) Veröffentlichungstag und Bekanntmachung des Hinweises auf die Patenterteilung: **14.04.2021**
(21) Anmeldenummer: 17720044.1
(22) Anmeldetag: 20.04.2017
(51) Int. Cl.: A61K 8/97, A61K 8/02, A61K 8/06, A61K 8/26, A61Q 19/00

(54) **ÖL-IN-WASSER-EMULSIONEN MIT EINEM GEHALT AN ISOFLAVONE ENTHALTENDEN PFLANZENEXTRAKTEN UND EINEM ODER MEHREREN GLIMMERPIGMENTEN**
OIL-IN-WATER EMULSIONS WITH ISOFLAVONE CONTAINING PLANT EXTRACTS AND MICA PIGMENTS
EMULSIONS HUILE DANS EAU AVEC DES EXTRAITS DE PLANTE COMPRENANT DES ISOFLAVONES ET DES PIGMENTS MICA

(30) Priorität: 13.05.2016 DE 102016208256
(43) Veröffentlichungstag der Anmeldung: 20.03.2019
(73) Patentinhaber: Beiersdorf AG, 20253 Hamburg (DE)
(72) Erfinder: FEY, Sven, 22397 Hamburg (DE); BOELKE, Dana, 22297 Hamburg (DE); MOEBIUS, Janne, 20257 Hamburg (DE)
(86) Internationale Anmeldenummer: PCT/EP2017/059353
(87) Internationale Veröffentlichungsnummer: WO 2017/194284

(56) Entgegenhaltungen:
- EP-A1- 2 545 898
- WO-A1-2015/135016
- DATABASE GNPD [Online] MINTEL; Juli 2011 (2011-07), "Day Care for Dry Skin", XP002770681, Database accession no. 1600226

## Beschreibung

Die vorliegende Erfindung betrifft stabilisierte kosmetische und dermatologische Formulierungen mit einem Gehalt an einem oder mehreren Pflanzenextrakten sowie einem oder mehreren Mikropigmenten.

Das Problem "sensible Haut" betrifft eine wachsende Anzahl Erwachsener und Kinder. Mit sensibler Haut bezeichnet man eine Kombination verschiedener Symptome, wie hyperreaktive und intolerante Haut. Aber auch atopische Haut kann darunter subsummiert werden. Diese Hautzustände werden von den Betroffenen oftmals, nicht ganz korrekt, als "allergische" Haut bezeichnet. Obwohl eine allergische Erkrankung zu Symptomen der sensiblen Haut führen kann, ist die Erscheinung "sensible Haut" nicht auf Allergiker beschränkt.

Die Haut, insbesondere die Epidermis, ist als Barriereorgan des menschlichen Organismus in besonderem Maße äußeren Einwirkungen unterworfen. Nach dem heutigen wissenschaftlichen Verständnis repräsentiert die Haut ein immunologisches Organ, das als immunkompetentes peripheres Kompartiment eine eigene Rolle in induktiven, effektiven und regulativen Immunprozessen des Gesamtorganismus spielt.

Die Epidermis ist reich mit Nerven und Nervenendapparaten wie Vater-Pacini-Lamellenkörpern, Merkel-Zell-Neuritenkomplexen und freien Nervenendigungen für Schmerz-, Kälte-, Wärmeempfindung und Juckreiz ausgestattet.

Bei Menschen mit sensibler, empfindlicher oder verletzlicher Haut kann ein mit "Stinging" (<engl.> "to sting" = verletzen, brennen, schmerzen) bezeichnetes neurosensorisches Phänomen beobachtet werden. Diese "sensible Haut" unterscheidet sich grundsätzlich von "trockener Haut" mit verdickten und verhärteten Hornschichten.

Typische Reaktionen des "Stinging" bei sensibler Haut sind Rötung, Spannen und Brennen der Haut sowie Juckreiz.

Bei atopischer Haut ist der Juckreiz als neurosensorisches Phänomen anzusehen. "Stinging"-Phänomene können als kosmetisch zu behandelnde Störungen angesehen werden. Starker Juckreiz dagegen, insbesondere bei Atopie auftretendes starkes Hautjucken, kann auch als schwerwiegendere dermatologische Störung bezeichnet werden.

Typische, mit den Begriffen "Stinging" oder "empfindlicher Haut" in Verbindung gebrachte, störende neurosensorische Phänomene sind Hautrötung, Kribbeln, Prickeln, Spannen und Brennen der Haut und Juckreiz. Sie können durch stimulierende Umgebungsbedingungen z.B. Massage, Tensideinwirkung, Wettereinfluss wie Sonne, Kälte, Trockenheit, aber auch feuchte Wärme, Wärmestrahlung und UV-Strahlung, z.B. der Sonne, hervorgerufen werden.

Nach bisherigen Erkenntnissen tritt eine derartige Empfindlichkeit gegenüber ganz bestimmten Substanzen individuell unterschiedlich auf. Dies bedeutet, eine Person, die bei Kontakt mit einer Substanz "Stingingeffekte" erlebt, wird sie mit hoher Wahrscheinlichkeit bei jedem weiteren Kontakt wiederholt erleben. Der Kontakt mit anderen "Stingern" kann aber ebensogut ohne jede Reaktion verlaufen.

Viele mehr oder weniger empfindliche Personen haben auch bei Verwendung mancher desodorierenden oder antitranspirierend wirkenden Zubereitungen unter erythematösen Hauterscheinungen zu leiden.

Erythematöse Hauterscheinungen treten auch als Begleiterscheinungen bei gewissen Hauterkrankungen oder -unregelmäßigkeiten auf. Beispielsweise ist der typische Hautausschlag beim Erscheinungsbild der Akne regelmäßig mehr oder weniger stark gerötet.

Daher sollten insbesondere Wirkstoffe und Zubereitungen, solche Wirkstoffe enthaltend, zur kosmetischen und dermatologischen Behandlung und/oder Prophylaxe erythematöser, entzündlicher, allergischer oder autoimmunreaktiver Erscheinungen, ins-besondere Dermatosen, aber auch des Erscheinungsbildes des "Stingings" zur Verfügung gestellt werden, die solche erythematösen Hauterscheinungen lindern

In dieser Hinsicht zum Beispiel beschreibt EP 2 545 898 A1 kosmetische Zusammensetzungen, welche die humane Haut vor Infrarotstrahlung schützen. Insbesondere werden kosmetische Zusammensetzungen offenbart mit Partikeln, wobei Mica (Glimmer) in einer Partikelgröße von 3-10 µm enthalten ist, sowie diverse Vitamine und Pflanzenextrakte. Einer dieser Extrakte ist aus grünen Kaffeebohnen.

Sojakeimextrakt ist bekanntermaßen ein antimikrobiell wirksames Agens und wurde seit Langem in der ostasiatischen Volksmedizin verwendet. Nachteilig ist, dass solche Extrakte leichtverderblich sind und sich bereits in kurzer Frist durch Lichteinfall, Luftzutritt und Temperatureinflüsse dunkel zu verfärben beginnen, wodurch ihre Wirksamkeit messbar abnimmt.

Eine weitere Aufgabe der vorliegenden Erfindung bestand daher darin, Wirkstoffe bzw. Zubereitungen, solche Wirkstoffe enthaltend, zur Verfügung zu stellen, die sich durch eine erhöhte Beständigkeit gegenüber Licht- und/oder Lufteinwirkung und/oder Temperatureinflüssen auszeichnen würden.

Die Verwendung von Effektpigmenten in der Kosmetik ist wohlbekannt. Ihre Erscheinung beruht auf Transmission und Reflexion des Lichtes an dem blattwerkartig orientierten Glimmerschuppenpigment.

Glimmer (INCI-Bezeichnung Mica) stellen zu den Phyllosilicaten gehörende, nach einer Fläche ausgezeichnet in dünne, elastische biegsame Blättchen spaltbare Hydroxy- und Alkalimetall-haltige Tonerde-Silicate der allgemeinen Formel

W(X,Y)₂₋₃[(OH,F)₂/Z₄O₁₀]

dar, mit W = K, Na oder Ca, seltener auch Ba, Rb und Cs; (X,Y) = je zwei Ionen von Al, Mg und/oder Fe oder Li, seltener auch Mn, Cr und Ti; Z = gewöhnlich Si und Al, aber auch Fe oder Ti. Zu den wichtigsten Glimmern mit chemischen Formeln, Dichte und Farben siehe die Tabelle. Am häufigsten sind Muscovit (Kaliglimmer) und Biotit (Magnesiaeisenglimmer), wobei die Anforderungen an Muscovit als Füllstoff für Beschichtungsstoffe in der DIN EN ISO 3262-12 beschrieben sind.

Die Glimmer kristallisieren überwiegend als tafelige bis kurzsäulige, meist sechsseitige (pseudohexagonale), zum Teil "Glimmer-Bücher" bildende Kristalle oder als unregelmäßig begrenzte, auf Spaltflächen perlmuttartig glänzende Blättchen oder Schuppen.

Man spricht von Perlglanzpigmenten, wenn die Beschichtungen ein irisierendes, perlmuttartiges Aussehen aufweisen. Dieses auf Interferenzphänomene sich gründende Aussehen der Beschichtungen rührt daher, dass die Glimmersubstrate mit Material belegt sind, dessen Brechzahl sich stark von der des Glimmers und des Bindemittels unterscheidet. Nicht beschichtetes Glimmerpigment zeigt Perlglanz-Effekte normalerweise nicht, da Glimmer eine sehr ähnliche Brechzahl wie Ölkomponenten aufweist (ca. 1,5) und sich somit optisch von denselben nicht abhebt. Erst durch Aufbringen von Überzügen mit einer anderen, i.a.höheren Brechzahl gelingt es, das Glimmerpigment optisch in Erscheinung treten zu lassen. Besonders bekannt geworden sind in der Vergangenheit Überzüge aus TiO₂, ZrO₂, Fe₂O₃, und ihre Hydroxide sowie Kombinationen derselben. So weisen beispielsweise die in DBP 14 67 468 und US 4 146 403 beschriebenen Perlglanzpigmente eine aus zwei Komponenten bestehende transparente Beschichtung auf, nämlich zunächst eine erste aus TiO₂ oder ZrO₂ bestehende farblose Schicht und darüber eine aus FeOOH, Fe₂O₃, Cr₂O₃, oder V₃O₅ bestehende farbige Schicht.

Es wurde überraschend gefunden, und darin liegt die Lösung all dieser Aufgaben, dass kosmetische oder dermatologische Öl-in-Wasser-Emulsionen mit einem Gehalt an
a) einem oder mehreren ein oder mehrere Isoflavone enthaltenden Sojakeimextrakt
   und
b) einer oder mehreren Substanzen, gewählt aus der Gruppe der
   i) natürlichen Glimmer mit einer durchschnittlichen Teilchengröße von 1 - 300 µm
   ii) Glimmer, beschichtet mit Titandioxid einer durchschnittliche Teilchengröße von 1 - 300 µm
   iii) Glimmer, beschichtet mit Silica einer durchschnittliche Teilchengröße von 1 - 300 µm
   iv) Glimmer, beschichtet mit Titandioxid und Silica einer durchschnittliche Teilchengröße von 1 - 300 µm
   v) Glimmer, beschichtet mit Eisenoxid einer durchschnittliche Teilchengröße von 1 - 300 µm
   vi) Synthetischer Glimmer, beschichtet mit Titandioxid und Zinnoxid einer durchschnittlichen Teilchengröße von 1 - 300 µm
   den Nachteilen des Standes der Technik abhelfen Dabei beträgt erfindungsgemäß die Konzentration des enthaltenen Sojakeimextrakt 0,005 - 50,0 Gew.-%, insbesondere 0,01 - 20,0 Gew.-%, bevorzugt 0,02 - 10,0 Gew.-%, besonders bevorzugt 0,02 - 5,0 Gew.-%, ganz besonders vorteilhaft 0,05 - 3,0 Gew.-%, jeweils bezogen auf das Gesamtgewicht der Zusammensetzung. Z

Erstaunlicherweise sind erfindungsgemäße O/W-Emulsionen lagerstabil, und insbesondere die darin enthaltenen Sojakeimextrakte zeichnen sich eine stark erhöhte Lagerbeständigkeit aus.

Isoflavone sind zu den Flavonoiden zählende, gelegentlich auch als Isoflavonoide bezeichnete Gruppe, von meist gelblich gefärbten Pflanzenfarbstoffen, die sich von Isoflavon ableiten. Der unsubstituierte Grundkörper, das eigentliche Isoflavon (3-Phenylchromon, 3-Phenyl-4H-1-benzopyran-4-on) kommt in Kleearten vor.

Einige bekanntere Isoflavone sind Daidzein (4',7-Dihydroxy-Isoflavon), als 7-O-Glucosid Daidzin in Sojamehl; Genistein (4',5,7-Trihydroxy-Isoflavon) aus Sojabohnen und Rotklee; Prunetin (4',5-Dihydroxy-7-methoxy-Isoflavon) aus der Rinde von Pflaumenbäumen; Biochanin A (5,7-Dihydroxy-4'-methoxy-Isoflavon) aus Kichererbsen, Rotklee und anderen Kleearten; Orobol (3',4',5,7-Tetrahydroxy-Isoflavon); Santal (3',4',5-Trihydroxy-7-methoxy-Isoflavon) aus Sandelholz, Rotholz und anderen Hölzern; Pratenseïn (3',5,7-Trihydroxy-4'-methoxyisoflavon) aus frischem Rot- oder Wiesenklee. Einige dieser in Kleearten und Leguminosen wie Luzerne vorkommenden Isoflavone zeigen Östrogenwirkung auf Weidetiere und können unter umständen zu Fortpflanzungsstörungen bei diesen führen.

Im Folgenden sind Substitutionsschemata einiger natürlich vorkommender Isoflavone aufgeführt:

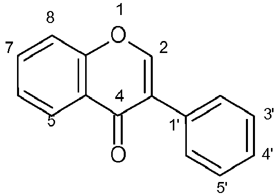

| | 5 | 7 | 3' | 4' | CAS-Nr. |
|---|---|---|---|---|---|
| Isoflavon | H | H | H | H | 574-12-9 |
| Daidzeïn | H | OH | H | OH | 486-68-8 |
| Genistein | OH | OH | H | OH | 446-72-0 |
| Genistin | OH | OH | H | OH | 446-72-0 |
| Prunetin | OH | OCH 3 | H | OH | 552-59-0 |
| Biochanin A | OH | OH | H | OCH3 | 491-80-5 |
| Orobol | OH | OH | OH | OH | 480-23-9 |
| Santal | OH | OCH 3 | OH | OH | 529-60-2 |
| Pratenseïn | OH | OH | OH | OCH3 | 2284-31-3 |

Als erfindungsgemäß einzusetzende Isoflavone kommen vorzugsweise die in der vorstehenden Tabelle aufgeführten Isoflavone in Betracht.

Von diesen wiederum ist bevorzugt das Genistein.

Genistein, 5,7-Dihydroxy-3-(4-hydroxyphenyl)-4H-1-benzopyran-4-on, 4',5,7-Trihydroxyisoflavon, auch Differenol A, Prunetol und Sophoricol genannt. weist folgende Struktur auf:

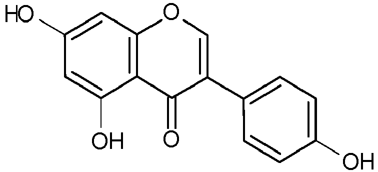

Genistein ist ein Sekundärmetabolit aus Pflanzen (Leguminosen, Papilionoiden, Rosaceen), wurde jedoch auch in Kulturen von Mikroorganismen (Actinomyceten, Aspergillus, Mycobacterien) gefunden. Es wurden schwach östrogene und antibakterielle Wirkungen besch rieben.

Bevorzugt enthalten kosmetische oder dermatologische Zubereitungen gemäß der Erfindung 0,001 - 10 Gew.-%, besonders bevorzugt 0,01 - 1 Gew.-%, an einem oder mehreren Isoflavonoiden, bezogen auf die Gesamtzusammensetzung der Zubereitungen.

Bevorzugt werden die Gewichtsverhältnisse von einem oder mehreren Isoflavonoiden zu einem oder mehreren Biochinonen in den Wirkstoffkombinationen und Zubereitungen, solche Wirkstoffzubereitungen enthaltend, gewählt aus dem Bereich von 50 : 1 bis 1 : 50, vorteilhaft aus dem Bereich von 10 : 1 bis 1 : 50, insbesondere vorteilhaft 2 : 1 bis 1 :2. Insbesondere ist es dabei vorteilhaft, wenn sich diese Extrakte zusätzlich durch einen Gehalt an Saponinen auszeichnen, da die zu erzielenden Wirkungen in diesem Fall besonders ausgeprägt sind. Der Gehalt an Saponinen sollte dabei in einem Konzentrationsbereich von 0.001 - 2 Gew.-%, bevorzugt 0,02 - 0.04 Gew.-%, bezogen auf die Gesamtzusammensetzung der kosmetischen Zubereitung, liegen.

Dabei kann eine vorteilhafte Ausführungsform der vorliegenden Erfindung darin bestehen, dass das oder die Isoflavonoide als Isoflavonoid-haltiger Extrakt in Form des Soja-Extraktes eingesetzt wird oder werden, der neben Isoflavonoiden, bevorzugt Genistein, 5 - 20 Gew.-% Saponine, besonders vorteilhaft 10 - 18 Gew.-% Saponine, jeweils bezogen auf das Gesamtgewicht des Extraktes, enthält.

Erfindungsgemäß vorteilhafte Isoflavonextrakte sind dadurch gekennzeichnet, dass der pulverförmige Isoflavonextrakt dergestalt vorliegt, dass mindestens 65 Gewichts-% der Teilchen eine Korngröße von kleiner 30 µm aufweisen. Dabei ist es erfindungsgemäß bevorzugt, wenn mindestens 70 % der Teilchen eine Korngröße von kleiner 30 µm aufweisen. Erfindungsgemäß bevorzugt können Extrakte eingesetzt werden, die nach der INCI mit Soybean (Glycine Soya) Germ Extract deklariert werden.

Erfindungsgemäß bevorzugt sind beispielsweise Isoflavonextrakte, die unter dem Handelsnamen "Isoflavones 150" und "Isoflavones Micro" bei Lucas Meyer Cosmetics S.A. erhältlich sind.

Die Herstellung des verwendeten Extraktes aus der Droge erfolgt mit Hilfe von superkritischem CO₂ oder Ethanol oder einer Ethanol / Wasser Mischung.

Als Wirkkomponenten und Leitsubstanzen des Extraktes sind Isoflavone beschrieben. Erfindungsgemäß enthalten kosmetische oder dermatologische Zubereitungen einen oder mehrere ein oder mehrere Isoflavone enthaltenden Sojakeimextrakt - entsprechend einem Gehalt von 0,005 - 50,0 Gew.-%, insbesondere 0,01 - 20,0 Gew.-%, bevorzugt 0,02 - 10,0 Gew.-%, besonders bevorzugt 0,02 - 5,0 Gew, ganz besonders vorteilhaft 0,05 - 3,0 Gew.-%, jeweils bezogen auf das Gesamtgewicht der Zusammensetzung.

Bevorzugt enthalten kosmetische oder dermatologische Zubereitungen gemäß der Erfindung eine oder mehrere Substanzen, gewählt aus der Gruppe der
i) natürlichen Glimmer mit einer durchschnittlichen Teilchengröße von 1 - 300 µm
ii) Glimmer, beschichtet mit Titandioxid einer durchschnittliche Teilchengröße von 1 - 300 µm
iii) Glimmer, beschichtet mit Silica einer durchschnittliche Teilchengröße von 1 - 300 µm
iv) Glimmer, beschichtet mit Titandioxid und Silica einer durchschnittliche Teilchengröße von 1 - 300 µm
v) Glimmer, beschichtet mit Eisenoxid einer durchschnittliche Teilchengröße von 1 - 300 µm
vi) Synthetischer Glimmer, beschichtet mit Titandioxid und Zinnoxid einer durschnittlichen Teilchengröße von 1 - 300 µm
entsprechend einem Gehalt von 0,005 - 50,0 Gew.-%, insbesondere 0,01 - 20,0 Gew.-%, bevorzugt 0,02 - 10,0 Gew.-%, besonders bevorzugt 0,02 - 5,0 Gew, ganz besonders vorteilhaft 0,05 - 3,0 Gew.-%, jeweils bezogen auf das Gesamtgewicht der Zusammensetzung.

Vorteilhafte Glimmerpigmente sind beispielsweise folgende Handelsprodukte:
Timiron® Super Silk MP 1005 (Partikelgröße < 15 µm. INCI: Mica + Titanium Dioxide.
Timiron® Super Sheen MP 1001 (Partikelgröße 5 - 25 µm. INCI: Mica + Titanium Dioxide.
Timiron® Arctic Silver (Partikelgröße 10 - 60 µm. INCI: Mica + Titanium Dioxide + Silica
Timiron® Synwhite 40 (Partikelgröße 5 - 40 µm. INCI: Synthetic Fluorphlogopite + Titanium Dioxide + Tion Oxide

Erfindungsgemäße, vorteilhaft als Emulsionen vorliegende Zubereitungen enthalten eine oder mehrere ober- oder grenzflächenaktiven polyethoxylierten Verbindungen, die beispielsweise vorteilhaft gewählt werden können aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel R-O-(-CH₂-CH₂-O-)ₙ-R',
- der Fettsäureethoxylate der allgemeinen Formel R-COO-(-CH₂-CH₂-O-), -H,
- der veretherten Fettsäureethoxylate der allgemeinen Formel R-COO-(-CH₂-CH₂-O-)ₙ -R',
- der veresterten Fettsäureethoxylate der allgemeinen Formel R-COO-(-CH₂-CH₂-O-)ₙ -C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel R-O-(-CH₂-CH₂-O-)ₙ-CH₂-COOH nd n eine Zahl von 5 bis 30 darstellen,
- der Polyoxyethylensorbitolfettsäureester,
- der Alkylethersulfate der allgemeinen Formel R-O-(-CH₂-CH₂-O-)ₙ-SO₃-H
- der Fettalkoholpropoxylate der allgemeinen Formel R-O-(-CH₂-CH(CH₃)-O-)ₙ-H,
- der Polypropylenglycolether der allgemeinen Formel R-O-(-CH₂-CH(CH₃)-O-)ₙ-R',
- der propoxylierten Wollwachsalkohole,
- der veretherten Fettsäurepropoxylate R-COO-(-CH₂-CH(CH₃)-O-)ₙ-R',
- der veresterten Fettsäurepropoxylate der allgemeinen Formel R-COO-(-CH₂-CH(CH₃)-O-)ₙ-C(O)-R',
- der Fettsäurepropoxylate der allgemeinen Formel R-COO-(-CH₂-CH(CH₃)-O-)ₙ-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel R-O-(-CH₂-CH(CH₃)O-)ₙ-CH₂-COOH
- der Alkylethersulfate bzw. die diesen Sulfaten zugrundeliegenden Säuren der allgemeinen Formel R-O-(-CH₂-CH(CH₃)-O-)ₙ-SO₃-H
- der Fettalkoholethoxylate/propoxylate der allgemeinen Formel R-O-Xₙ-Yₘ-H,
- der Polypropylenglycolether der allgemeinen Formel R-O-Xₙ-Yₘ-R',
- der veretherten Fettsäurepropoxylate der allgemeinen Formel R-COO-Xₙ-Yₘ-R',
- der Fettsäureethoxylate/propoxylate der allgemeinen Formel R-COO-Xₙ-Yₘ-H,.

Erfindungsgemäß besonders vorteilhaft werden die eingesetzten ober- oder grenzflächenaktiven polyethoxylierten Verbindungen gewählt aus der Gruppe der Substanzen mit HLB-Werten von 11 - 18, ganz besonders vorteilhaft mit mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(2)stearylether (Steareth-2), Polyethylenglycol(14)stearylether (Steareth-14), Polyethylenglycol(16)stearylether (Steareth-16), Polyethylenglycol(17)stearylether (Steareth-17), Polyethylenglycol(18)stearylether (Steareth-18), Polyethylenglycol(19)stearylether (Steareth-19), Polyethylenglycol(20)stearylether (Steareth-20), Polyethylenglycol-(21 )stearylether (Steareth-21),
Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol-(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol-(20)isostearylether (Isosteareth-20),
Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),
Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)isocetylether (Isoceteth-17), Polyethylenglycol-(18)isocetylether (Isoceteth-18), Polyethylenglycol(19)isocetylether (Isoceteth-19), Polyethylenglycol(20)isocetylether (Isoceteth-20),
Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15),
Polyethylenglycol(12)laurylether (Laureth-12), Polyethylenglycol(12)isolaurylether (Isolaureth-12).
Polyethylenglycol(13)cetylstearylether (Ceteareth-13), Polyethylenglycol(14)cetylstearyl-ether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol(19)cetyl-stearylether (Ceteareth-19), Polyethylenglycol(20)cetylstearylether (Ceteareth-20).

Es ist ferner von Vorteil, die Fettsäureethoxylate aus folgender Gruppe zu wählen:
Polyethylenglycol(8)stearat, Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat, Polyethylenglycol(30)stearat, Polyethylenglycol(40)stearat, Polyethylenglycol(100)stearat, Polyethylenglycol(200)stearat, Polyethylenglycol(400)stearat,
Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol-(17)isostearat, Polyethylenglycol(18)isostearat, Polyethylenglycol(19)isostearat, Polyethylenglycol(20)isostearat, Polyethylenglycol(21 )isostearat, Polyethylenglycol(22)isostearat, Polyethylenglycol(23)isostearat, Polyethylenglycol(24)isostearat, Polyethylenglycol(25)isostearat, Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat.

Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Ebenfalls vorteilhaft sind ethoxylierte Rizinusölverbindungen wie Polyethylenglycol(40) hydriertes Rizinusöl (INCI: Polyethylenglycol(40) hydrogenated Castor oil).

Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21 )glyceryllaurat, Polyethylenglycol(22)-glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Bevorzugte ober- oder grenzflächenaktive polyethoxylierten Verbindungen im Sinne der vorliegenden Erfindung sind Polyethylenglycol(8)stearat, Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat, Polyethylenglycol(30)stearat, Polyethylenglycol(40)stearat, Polyethylenglycol(100)stearat, Polyethylenglycol(200)stearat, Polyethylenglycol(400)stearat, Polyethylenglycol(2)stearylether, Polyethylenglycol(21 )stearylether, Polyethylenglycol(20)cetylstearylether, Polyethylenglycol(40) hydriertes Rizinusöl, Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat.

Die erfindungsgemäß verwendeten Wirkstoffkombinationen lassen sich ohne Schwierigkeiten in gängige kosmetische oder dermatologische Formulierungen einarbeiten, vorteilhaft in Pumpsprays, Aerosolsprays, Cremes, Salben, Tinkturen, Lotionen, Nagelpflegeprodukte (z.B. Nagellacke, Nagellackentferner, Nagelbalsame) und dergleichen.

Es ist auch gegebenenfalls vorteilhaft, die Zusammensetzungen gemäß der Erfindung abzupuffern. Vorteilhaft ist ein pH-Bereich von 4,5 - 8,5. Besonders günstig ist es, den pH-Wert in einem Bereich von 6,0 - 8,0 zu wählen.

Die kosmetischen und/oder dermatologischen Formulierungen gemäß der Erfindung können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Zur Anwendung werden die kosmetischen und/oder dermatologischen Formulierungen gemäß der Erfindung in der für Kosmetika und Dermatika üblichen Weise auf die Haut und/oder die Haare in ausreichender Menge aufgebracht.

Vorteilhaft sind solche kosmetischen und dermatologischen Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Kosmetische Zubereitungen gemäß der Erfindung zum Schutze der Haut vor UV-Strahlen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-ÖI-in-Wasser (W/O/W), ein Gel, eine Hydrodispersion, einen festen Stift oder auch ein Aerosol darstellen.

Die kosmetischen Zubereitungen gemäß der Erfindung können kosmetische Hilfsstoffe enthalten, wie sie üblicherweise in solchen Zubereitungen verwendet werden, z.B. Konservierungsmittel, Bakterizide, Antioxidantien, Parfüme, Mittel zum Verhindern des Schäumens, Farbstoffe, Pigmente, die eine färbende Wirkung haben, Verdikkungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Emulsionen sind gemäß der Erfindung vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wässrig-alkoholischen oder alkoholischen Gelen vorzugweise ein Polyacrylat ist.

Feste Stifte gemäß der Erfindung enthalten z.B. natürliche oder synthetische Wachse, Fettalkohole oder Fettsäureester. Bevorzugt werden Lippenpflegestifte sowie desodorierende Stifte ("Deo-Sticks").

Als Treibmittel für erfindungsgemäße, aus Aerosolbehältern versprühbare kosmetische oder dermatologische Zubereitungen sind die üblichen bekannten leichtflüchtigen, verflüssigten Treibmittel, z.B. Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können die erfindungsgemäßen Zubereitungen zudem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen.

Bei kosmetischen Zubereitungen zur Pflege der Haare handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen Zubereitungen enthalten Wirkstoffe und Hilfsstoffe, wie sie üblicherweise für diesen Typ von Zubereitungen zur Haarpflege und Haarbehandlung verwendet werden.

Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die Zubereitung selbst zu färben, Elektrolyte, Zubereitungen gegen das Fetten der Haare.

Kosmetische Zubereitungen, die ein Shampoonierungsmittel oder eine Wasch-, Dusch- oder Badezubereitung darstellen, enthalten vorzugsweise mindestens eine anionische, nichtionische oder amphotere oberflächenaktive Substanz oder Gemische daraus, mindestens eine dialkylsubstituierte Carbonsäure im wäßrigen Medium und Hilfsmittel, wie sie üblicherweise dafür verwendet werden.

Die folgenden Beispiele sollen die Verkörperungen der vorliegenden Erfindungen verdeutlichen. Die Angaben beziehen sich stets auf Gewichts-%, sofern nicht andere Angaben gemacht werden.

| **Beispielrezepturen** : | **Variante 1** | **Variante 2** | **Variante 3** | **Variante 4** | **Variante 5** |
|---|---|---|---|---|---|
| **INCI** | **m [%]** | **m [%]** | **m [%]** | **m [%]** | **m [%]** |
| | | | | | |
| Phenoxyethanol | 0,6 | 0,6 | 0,6 | 0,6 | 0,6 |
| Cyclomethicone | 15 | 5 | 8 | 8 | 12 |
| Cyclomethicone + Dimethiconol | 15 | 5 | 8 | 7 | 13 |
| Methylpropanediol | 2 | 2 | 2 | 2 | 2 |
| Polymethylsilsesquioxane | 10 | 4 | 8 | 8 | 12 |
| 1,2-Hexanediol | 0,5 | 0,5 | 0,5 | 0,5 | 0,5 |
| Polyglyceryl-3 Methylglucose Distearate | 0,5 | 1 | 1,5 | 1 | 2 |
| Chondrus Crispus Powder | 0,3 | 0,2 | 0,4 | 0,25 | 0,15 |
| Algin | 0,5 | 0,6 | 0,7 | 0,8 | 0,65 |
| Parfum | 0,49 | 0,6 | 0,82 | 0,49 | 0,82 |
| Glycine Soja Germ Extract | 0,05 | 0,5 | 0,1 | 1 | 0,75 |
| Synthetic Fluorphlogopite+Cl 77891+Tin Oxide | 1 | 2 | 0,5 | 0,75 | 1 |
| **Wasser:** | **Ad 100,00** | | | | |

## Patentansprüche

1. Kosmetische oder dermatologische Öl-in-Wasser-Emulsionen mit einem Gehalt an
a) einem oder mehreren ein oder mehrere Isoflavone enthaltenden Sojakeimextrakt, wobei dessen Konzentration 0,005 - 50,0 Gew.-%, insbesondere 0,01 - 20,0 Gew.-%, bevorzugt 0,02 - 10,0 Gew.-%, besonders bevorzugt 0,02 - 5,0 Gew, ganz besonders vorteilhaft 0,05 - 3,0 Gew.-% beträgt, jeweils bezogen auf das Gesamtgewicht der Zusammensetzung.
und
b) einer oder mehreren Substanzen, gewählt aus der Gruppe der
i) natürlichen Glimmer mit einer durchschnittlichen Teilchengröße von 1 - 300 µm
ii) Glimmer, beschichtet mit Titandioxid einer durchschnittliche Teilchengröße von 1 - 300 µm
iii) Glimmer, beschichtet mit Silica einer durchschnittliche Teilchengröße von 1 - 300 µm
iv) Glimmer, beschichtet mit Titandioxid und Silica einer durchschnittliche Teilchengröße von 1 - 300 µm
v) Glimmer, beschichtet mit Eisenoxid einer durchschnittliche Teilchengröße von 1 - 300 µm
vi) Synthetischer Glimmer, beschichtet mit Titandioxid und Zinnoxid einer durchschnittlichen Teilchengröße von 1 - 300 µm

2. Zubereitungen gemäß Anspruch 1, enthaltend eine oder mehrere Substanzen, gewählt aus der Gruppe der
i) Natürlichen Glimmer mit einer durchschnittlichen Teilchengröße von 1 - 300 µm
ii) Glimmer, beschichtet mit Titandioxid einer durchschnittliche Teilchengröße von 1 - 300 µm
iii) Glimmer, beschichtet mit Silica einer durchschnittliche Teilchengröße von 1 - 300 µm
iv) Glimmer, beschichtet mit Titandioxid und Silica einer durchschnittliche Teilchengröße von 1 - 300 µm
v) Glimmer, beschichtet mit Eisenoxid einer durchschnittliche Teilchengröße von 1 - 300 µm
vi) Synthetischer Glimmer, beschichtet mit Titandioxid und Zinnoxid einer durschnittlichen Teilchengröße von 1 - 300 µm
entsprechend einem Gehalt von 0,005 - 50,0 Gew.-%, insbesondere 0,01 - 20,0 Gew.-%, bevorzugt 0,02 - 10,0 Gew.-%, besonders bevorzugt 0,02 - 5,0 Gew, ganz besonders vorteilhaft 0,05 - 3,0 Gew.-%, jeweils bezogen auf das Gesamtgewicht der Zusammensetzung.

## Claims

1. Cosmetic or dermatological oil-in-water emulsions having a content of
a) one or more soya germ extract containing one or more isoflavones, the concentration of which is 0.005 - 50.0% by weight, particularly 0.01 - 20.0% by weight, preferably 0.02 - 10.0% by weight, particularly preferably 0.02 - 5.0% by weight, especially advantageously 0.05 - 3.0% by weight, based in each case on the total weight of the composition
and
b) one or more substances selected from the group of
i) natural mica having a mean particle size of 1 - 300 µm
ii) mica coated with titanium dioxide of mean particle size of 1 - 300 µm
iii) mica coated with silica of mean particle size of 1 - 300 µm
iv) mica coated with titanium dioxide and silica of mean particle size of 1 - 300 µm
v) mica coated with iron oxide of mean particle size of 1 - 300 µm
vi) synthetic mica coated with titanium dioxide and tin oxide of mean particle size of 1 - 300 µm.

2. Preparations according to Claim 1, comprising one or more substances selected from the group comprising
i) natural mica having a mean particle size of 1 - 300 µm
ii) mica coated with titanium dioxide of mean particle size of 1 - 300 µm
iii) mica coated with silica of mean particle size of 1 - 300 µm
iv) mica coated with titanium dioxide and silica of mean particle size of 1 - 300 µm
v) mica coated with iron oxide of mean particle size of 1 - 300 µm
vi) synthetic mica coated with titanium dioxide and tin oxide of mean particle size of 1 - 300 µm.
corresponding to a content of 0.005 - 50.0% by weight, particularly 0.01 - 20.0% by weight, preferably 0.02 - 10.0% by weight, particularly preferably 0.02 - 5.0% by weight, especially advantageously 0.05 - 3.0% by weight, based in each case on the total weight of the composition.

## Revendications

1. Émulsions cosmétiques ou dermatologiques huile-dans-eau dotées d'une teneur en
a) un ou plusieurs extraits de germe de soja contenant une ou plusieurs isoflavones, dont la concentration est de 0,005 à 50,0 % en poids, en particulier 0,01 à 20,0 % en poids, préférablement 0,02 à 10,0 % en poids, particulièrement préférablement 0,02 à 5,0 % en poids, tout particulièrement avantageusement 0,05 à 3,0 % en poids, à chaque fois par rapport au poids total de la composition,
et
b) une ou plusieurs substances choisies dans le groupe
i) des micas naturels dotés d'une taille moyenne de particule de 1 à 300 µm,
ii) des micas, revêtus avec du dioxyde de titane d'une taille moyenne de particule de 1 à 300 µm,
iii) des micas, revêtus avec une silice d'une taille moyenne de particule de 1 à 300 µm,
iv) des micas, revêtus avec du dioxyde de titane et une silice d'une taille moyenne de particule de 1 à 300 µm,
v) des micas, revêtus avec un oxyde de fer d'une taille moyenne de particule de 1 à 300 µm
vi) des micas synthétiques, revêtus avec du dioxyde de titane et de l'oxyde d'étain d'une taille moyenne de particule de 1 à 300 µm.

2. Préparations selon la revendication 1, contenant une ou plusieurs substances, choisies dans le groupe
i) des micas naturels dotés d'une taille moyenne de particule de 1 à 300 µm,
ii) des micas, revêtus avec du dioxyde de titane d'une taille moyenne de particule de 1 à 300 µm,
iii) des micas, revêtus avec une silice d'une taille moyenne de particule de 1 à 300 µm,
iv) des micas, revêtus avec du dioxyde de titane et une silice d'une taille moyenne de particule de 1 à 300 µm,
v) des micas, revêtus avec un oxyde de fer d'une taille moyenne de particule de 1 à 300 µm
vi) des micas synthétiques, revêtus avec du dioxyde de titane et de l'oxyde d'étain d'une taille moyenne de particule de 1 à 300 µm,
correspondant à une teneur de 0,005 à 50,0 % en poids, en particulier 0,01 à 20,0 % en poids, préférablement 0,02 à 10,0 % en poids, particulièrement préférablement 0,02 à 5,0 % en poids, tout particulièrement avantageusement 0,05 à 3,0 % en poids, à chaque fois par rapport au poids total de la composition.
